# EUROPEAN PATENT APPLICATION

(11) **EP 2 710 975 A2**
(43) Date of publication of application: **26.03.2014**
(21) Application number: 13179462.0
(22) Date of filing: 06.08.2013
(51) Int. Cl.: A61B 19/00, A61N 7/00, A61N 7/02

(54) **Medical robot apparatus**

(30) Priority: 21.09.2012 KR 20120105242
(71) Applicant: Samsung Electronics Co., Ltd, Gyeonggi-do 443-742 (KR)
(72) Inventor: Seo, Joon-ho, Yongin-si, Gyeonggi-do (KR); Kim, Do-kyoon, Yongin-si, Gyeonggi-do (KR); Kim, Sang-hyun, Yongin-si, Gyeonggi-do (KR); Lee, Hee-sae, Yongin-si, Gyeonggi-do (KR); Bang, Won-chul, Yongin-si, Gyeonggi-do (KR)
(74) Representative: Bartelds, Erik

(57) **Abstract**

A medical apparatus includes a moving unit supported on the base and configured to move in a longitudinal direction of the bed. The moving unit is configured to extend in a width direction of the bed. The medical apparatus also includes a mount unit on the moving unit and configured to move in the width direction and including a medical applicator mounted thereon.

## Description

### BACKGROUND

### 1. Field

The present disclosure relates to medical robot apparatuses to perform examination and treatment by controlling medical applicators to access subjects lying on beds.

### 2. Description of the Related Art

Medical robot systems refer to robot systems that perform examination and/or treatment by enabling medical applicators to access patients' diseased parts. Medical robot systems have recently been used to perform precise examination/treatment through non-invasive energy transmission apparatuses or minimally invasive surgical tools.

A medical applicator used for examination and/or treatment is used in conjunction with a conventional medical robot system. Accordingly, in order to perform various examination and treatment operations, a plurality of medical robot systems, respectively, provided with various types of medical applicators are required, thereby increasing system costs and costs associated with the examination and/or treatment. A system including a unit moving a medical applicator and a bed, which are independent from each other, would occupy a large space, thereby being cumbersome system for a small hospital or a clinic.

### SUMMARY

This Summary is provided to introduce a selection of concepts in a simplified form that are further described below in the Detailed Description.

Provided are medical robot apparatuses having compact structures that may enable medical applicators to access subjects lying on beds.

Provided are medical robot apparatuses that may perform various medical operations in a limited space.

Provided are medical robot apparatuses that may enable medical applicators to access patients' diseased parts.

In accordance with an illustrative example, a medical apparatus includes a moving unit supported on a base and configured to move in a longitudinal direction of a bed on the base and extend in a width direction of the bed. The medical apparatus also includes a mount unit on the moving unit and configured to move in the width direction and including a medical applicator mounted thereon.

The mount unit may be configured to move through an arc shaped path of the moving unit in the width direction of the bed.

The moving unit may include a support unit supported on the base and configured to move in the longitudinal direction of the bed, and an extension unit extending in the width direction of the bed and configured to support the mount unit to move the mount unit in the width direction of the bed. The extension unit may be connected to the support unit and may pivot in the longitudinal direction of the bed.

The mount unit may be mounted on the extension unit to move in the width direction along an arc shaped movement path.

The mount unit may include a coupling unit coupling to the medical applicator, and a driver configured to drive the coupling unit with at least one degree of freedom.

The driver may be configured to move the coupling unit up and/or down relative to the moving unit.

The driver may be configured to rotate the coupling unit about a vertical axis.

The coupling unit may be connected to the driver via a ball joint unit.

The driver may include a first member rotatably coupled to a housing of the mount unit, and three linear actuators which are configured to couple the first member and the coupling unit and which are radially arranged. The three linear actuators may each be connected to the coupling unit via a ball joint unit.

The moving unit may be supported on the base and may be configured to move in the longitudinal direction along a movement path of which a portion is non-horizontal.

The movement path of the moving unit may include first, second and third paths. The first and second paths may correspond to an upper body and a lower body of a patient, respectively, and may be of different heights with respect to the bed. The third path may be inclined and may connect the first and second paths.

The driver may include a first gear configured on the first member, a rotary motor including a second gear engaged with the first gear, and a second member partly disposed in the first member, while the first member may be rotatably supported on a housing of the mount unit via a bearing.

When the rotary motor rotates, the first member may rotate about a vertical axis and a rotational force of the first member may be transmitted through a prismatic actuator to the second member to enable the second member to rotate.

The first member and the second member may rotate together, and the second member may rise and fall relative to the first member.

In accordance with another illustrative example, a medical apparatus includes a moving unit configured to extend in a width direction of a bed and includes a second driver configured to move a mount unit on which a medical applicator is mounted along an arc shaped movement path in the width direction. The medical apparatus includes a base including the bed and a first driver configured to move the moving unit in a longitudinal direction of the bed.

The moving unit may include a support unit configured to be moved by the first driver and an extension unit connected to the support unit and configured to enable the second driver to be disposed thereon. The extension unit may be connected to the support unit and may pivot in the longitudinal direction.

The mount unit may include a coupling unit configured to couple the medical applicator, and a third driver configured to drive the coupling unit with a one degree of freedom.

The third driver may move the coupling unit up in a radial direction of the arc shaped movement path, and the third driver may rotate the coupling unit about a vertical axis.

The first driver may move the moving unit in the longitudinal direction of the bed along a movement path of which a portion is non-horizontal.

The movement path of the moving unit may include first, second and third paths. The first and second paths may correspond to an upper body and a lower body of a patient, respectively, and may be of different heights with respect to the bed. The third path may be inclined and may connect the first and second paths.

According to the illustrative examples of the medical apparatus, it is possible to realize a compact medical apparatus which can perform various medical operations in a limited space. By providing the medical applicator with a plurality of degrees of freedom for movements, it is possible to realize a medical apparatus in which the medical applicator can easily access patients' diseased parts. Also, by moving the moving unit in the longitudinal direction of the bed along the movement path having a non-horizontal portion, it is possible to reduce a movement distance by which the medical applicator moves to access the patient's body part to be examines and/or treated and thereby, the medical applicator can easily access the body part to be examined and/or treated.

### BRIEF DESCRIPTION OF THE DRAWINGS

These and/or other aspects will become apparent and more readily appreciated from the following description of the embodiments, taken in conjunction with the accompanying drawings in which:
FIG. 1 is a front view illustrating a medical robot system for examination and/or treatment of a patient, according to an illustrative embodiment;
FIG. 2 is a side view illustrating an examination/treatment station, according to an illustrative embodiment;
FIG. 3 is a front view illustrating a first driving unit configured to move a moving unit in a longitudinal direction of a bed, according to an illustrative embodiment;
FIG. 4 is a perspective view illustrating a second driving unit configured to move a mount unit in a width direction, according to an illustrative embodiment;
FIG. 5A is a cross-sectional view illustrating a third driving unit configured to drive a medical applicator with at least 1 degree of freedom, according to an illustrative embodiment;
FIG. 5B is a plan view for explaining a connection between a first member and a second member of the third driving unit of FIG. 5A;
FIG. 6A is a cross-sectional view illustrating the third driving unit of FIG. 5A, according to another illustrative embodiment;
FIG. 6B is a perspective view to illustrate a connection between three prismatic actuators and a first member of the third driving unit of FIG. 6A;
FIG. 7A is a side view illustrating an examination/treatment station, according to another illustrative embodiment;
FIG. 7B is a side view illustrating a pivotal structure for pivoting an extension unit relative to a support unit, according to an illustrative embodiment;
FIG. 8 is a side view illustrating an examination/treatment station, according to another illustrative embodiment;
FIG. 9 is a cross-sectional view illustrating the first driving unit of the examination/treatment station of FIG. 8, according to an illustrative embodiment;
FIG. 10 is a cross-sectional view illustrating the first driving unit of the examination/treatment station of FIG. 8, according to another illustrative embodiment;
FIG. 11 is a cross-sectional view illustrating a coupling relationship between a rotational shaft of a guide roller and a connection member of FIG. 10;
FIG. 12 is a front view illustrating an examination/treatment station, according to another illustrative embodiment;
FIG. 13 is a side view illustrating an examination/treatment station, according to another illustrative embodiment; and
FIG. 14 is a perspective view illustrating a multi-joint surgical instrument as an example of the medical applicator, according to an illustrative embodiment.

### DETAILED DESCRIPTION

The following detailed description is provided to assist the reader in gaining a comprehensive understanding of the methods, apparatuses, and/or systems described herein. Accordingly, various changes, modifications, and equivalents of the systems, apparatuses and/or methods described herein will be suggested to those of ordinary skill in the art. Also, descriptions of well-known functions and constructions may be omitted for increased clarity and conciseness.

It will be understood that when an element or layer is referred to as being "on" or "connected to" or "coupled" to another element or layer, it can be directly on or connected to the other element or layer or through intervening elements or layers that may be present. In contrast, when an element is referred to as being "directly on" or "directly connected to" another element or layer, there are no intervening elements or layers present. Like reference numerals refer to like elements throughout. As used herein, the term "and/or" includes any and all combinations of one or more of the associated listed items. Wherever "a component" or "an element" is mentioned, this will be understood to describe one or more components or elements.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another region, layer or section. These terms do not necessarily imply a specific order or arrangement of the elements, components, regions, layers and/or sections. Thus, a first element, component, region, layer or section discussed below could be termed a second element, component, region, layer or section without departing from the teachings description of the present invention.

The units described herein may be implemented using hardware components and software components. For example, controllers, processors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

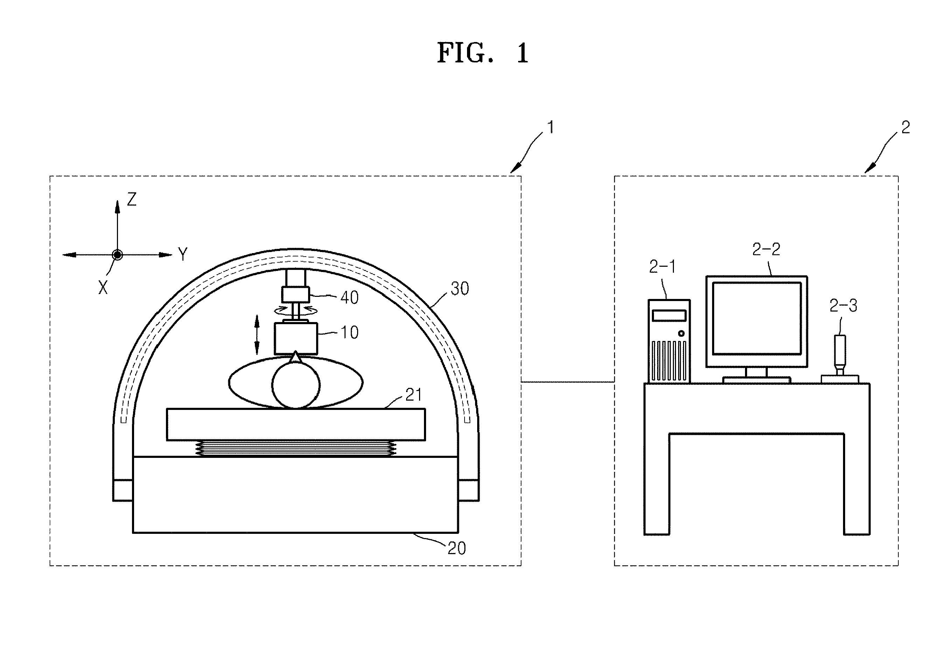

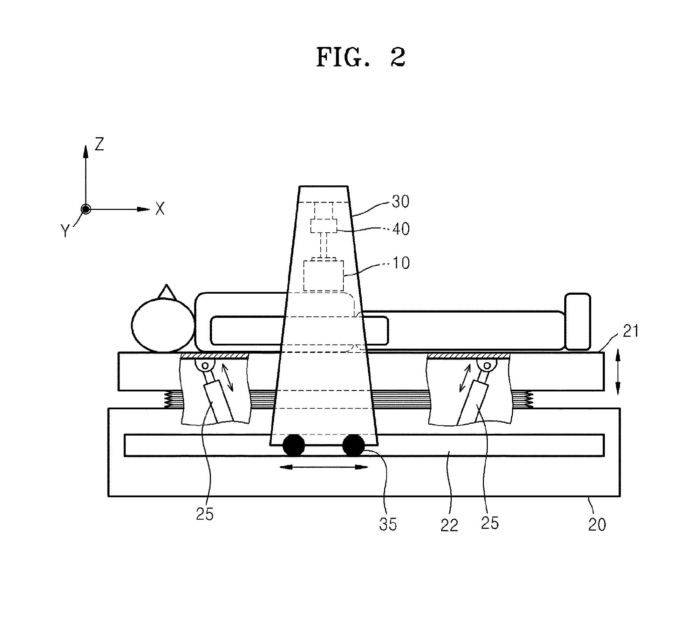

FIG. 1 is a front view illustrating a medical robot system for examination and/or treatment, according to an illustrative embodiment. FIG. 2 is a side view illustrating an examination/treatment station 1 of FIG. 1, according to an illustrative embodiment. Referring to FIGS. 1 and 2, the medical robot system performs examination or treatment by enabling a medical applicator 10 to access a subject, for example, a patient lying face up or down on a bed 21. The medical robot system includes an examination/treatment station 1 that includes mechanical devices to perform examination and/or treatment on the subject, and a control station 2 that controls the examination/treatment station 1.

The examination/treatment station 1 may include a base 20 that includes the bed 21, and a moving unit 30 that is supported on the base 20 and allows the medical applicator 10 to be provided thereon. With respect to the bed 21, the moving unit 30 moves in a longitudinal direction X and additionally extends upward and crosses a space above the bed 21 in a width direction Y. The medical applicator 10 may be coupled to the moving unit 30 to enable the medical applicator 10 to move in the width direction Y of the bed 21. For example, the moving unit 30 includes a mount unit 40 that moves in the width direction Y of the bed 21, and the medical applicator 10 coupled to the mount unit 40.

The control station 2 includes a controller 2-1 to control the examination/treatment station 1, a display 2-2, and a manipulation unit 2-3. A first driver to drive the moving unit 30, a second driver to drive the mount unit 40, and a third driver to drive the medical applicator 10 with at least 1 degree of freedom will be explained below and are connected to the controller 2-1. The display 2-2 displays an image of a diseased part for examination/treatment and an image showing an operating state of the examination/treatment station 1. An operator operates the manipulation unit 2-3 to control movements of the moving unit 30, the mount unit 40, and the medical applicator 10 to examine and/or treat a patient and may include at least one haptic manipulation device such as a joy stick. The operator may perform examination/treatment by operating the manipulation unit 2-3 to control the moving unit 30, the mount unit 40, and the medical applicator 10. Accordingly, in one illustrative example, the control station 2 is a device to control operations of the mechanical devices of the examination/treatment station 1.

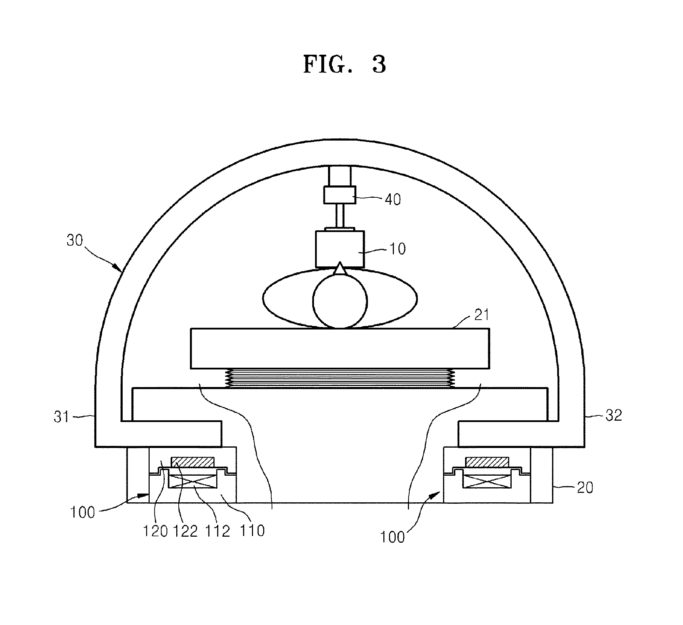

The moving unit 30 moves along a movement path that is in the longitudinal direction X and is parallel to the bed 21 as shown in FIG. 2. The first driver to move the moving unit 30 in the longitudinal direction X may be disposed on the base 20 and may include a linear motor 100. FIG. 3 is a front view illustrating the first driver to move the moving unit 30 in the longitudinal direction X of the bed 21, according to an illustrative embodiment. Although FIG. 3 illustrates two linear motors 100 at each end portion 31 and 32, for purposes of brevity only one linear motor 100 at end portion 31 will be described. However, it is to be appreciated that the linear motor 100 at the end portion 32 may be similarly configured as the linear motor 100 of the end portion 31. In one alternative configuration, one linear motor 100 may be implemented, either at the end portion 31 or at the end portion 32.

Referring to FIG. 3, the linear motor 100 includes a linear guide 110 that extends in the longitudinal direction X and is embedded with a coil 112. The linear motor 100 also includes a slider 120 that is mounted on the linear guide 110 and is embedded with a magnet 122. The slider 120 moves in the longitudinal direction X along the linear guide 110 as a result of an electromagnetic force generated between the coil 112 and the magnet 122. The end portion 31 and/or the other end portion 32 of the moving unit 30 may be coupled to the slider 120. In this structure, the moving unit 30 moves in the longitudinal direction X of the bed 21. Although not shown in FIG. 3, the linear motor 100 may further include controllers to detect a position and a speed of the slider 120, for example, a linear scale and an encoder. As shown in FIG. 2, the base 20 may include a guide slot 22 along which the moving unit 30 is guided. A guide roller 35 which is in, for example, rolling contact with the guide slot 22 may be provided on each of the one end portion 31 and the other end portion 32 of the moving unit 30.

The first driver moving the moving unit 30 in the longitudinal direction X is not limited to that shown in FIG. 3. For example, a belt or a wire which is driven by a motor may be provided in the longitudinal direction X of the bed 21. The moving unit 30 would move in the longitudinal direction X of the bed by connecting the one end portion 31 and/or the other end portion 32 of the moving unit 30 to the belt or the wire. In an alternative configuration, the first driver in which a lead screw is disposed in the longitudinal direction X of the bed 21 would mesh with the moving unit 30 through helical grooves of the lead screw. The moving unit 30 may move in the longitudinal direction X using a motor to rotate the lead screw.

The mount unit 40 moves through an arc shaped movement path provided on the moving unit 30, where the arc shaped movement path is curved upward above the bed 21. In this case, because the medical applicator 10 moves along the arc shaped movement path of the mount unit 40, the medical applicator 10 may easily access the patient's organ or part to be examined. However, a shape of the movement path of the mount unit 40 is not limited to an arc shape. The movement path may be configured to be any of various shapes as long as the configuration allows the medical applicator 10 to move in the width direction Y of the bed 21 to enable examination and/or treatment of the patient lying on the bed 21.

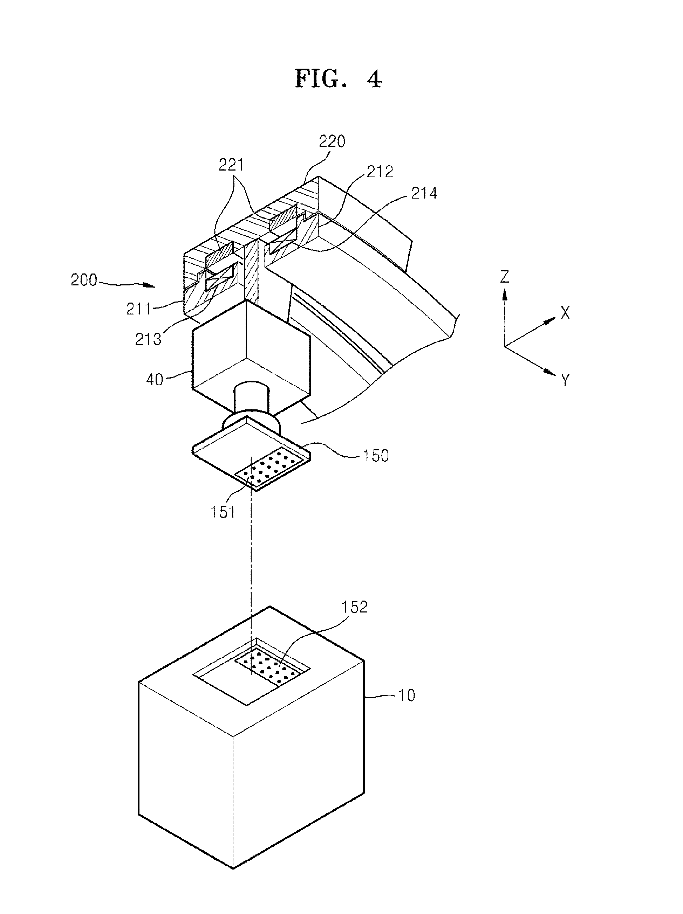

The second driver to move the mount unit 40 in the width direction Y may be provided on the moving unit 30. FIG. 4 is a perspective view illustrating the second driver to move the mount unit 40 along the arc shaped movement path, according to an illustrative embodiment. The second driver may include a linear motor 200 as shown in FIG. 4. For example, one pair of arc shaped linear guides 211 and 212 may be configured so that each of the arc shaped linear guides 211 and 212 are spaced apart from each other in the longitudinal direction X. A slider 220 is mounted on the linear guides 211 and 212. The slider 220 moves along the arc shaped linear guides 211 and 212 as a result of an electromagnetic force generated between magnets 221 embedded in the slider 220 and coils 213 and 214 embedded in the linear guides 211 and 212. The mount unit 40 is operatively connected or coupled to the slider 220 between the linear guides 211 and 212. Although not shown in FIG. 4, the linear motor 200 may further include at least one detector to detect a position and a speed of the slider 220, for example, a linear scale and an encoder.

The second driver is not limited to that shown in FIG. 4. For example, a belt or a wire that is driven along a substantially arc shaped path by a motor may be provided on the moving unit 30 in the width direction Y. The mount unit 40 may move in the width direction Y along the arc shaped movement path based on a connection to the belt or the wire.

The mount unit 40 includes a coupling unit 150 to which the medical applicator 10 is operatively connected or coupled. The coupling unit 150 may include any of various coupling structures to couple the medical applicator 10 thereto. For example, although not shown in FIG. 4, the medical applicator 10 may be coupled to the coupling unit 150 by using a magnetic force, a coupling member, complementary shapes, or a clamp.

An operation signal to operate the medical applicator 10 may be transmitted through a signal line that connects the medical applicator 10 and the control station 2. For example, the coupling unit 150 may include a first electrical contact unit 151 that is connected to the control station 2. The medical applicator 10 includes a second electrical contact unit 152. The second electrical contact unit 152 is operatively connected to the controller 2-1 of the control station 2 through the moving unit 30. Once the medical applicator 10 is coupled to the coupling unit 150, the first and second electrical contact units 151 and 152 are connected to each other, thereby enabling the control station 2 to control the medical applicator 10. In an alternative configuration, the medical applicator 10 and the control station 2 may be directly connected to each other by a signal line (not shown).

The bed 21 is placed on the base 20 to move up and/or down in a vertical direction Z. For example, the bed 21 may be connected to the base 20 by a linear actuator 25 which is an electric actuator, a hydraulic actuator, or a pneumatic actuator. A structure of the bed 21 is not limited thereto, and may be any of various structures, such as a structure using a lead screw and a collar having helical grooves meshing with the lead screw.

In the above configuration, the moving unit 30 can move in the longitudinal direction X, the mount unit 40 moves in the width direction Y, and the bed 21 moves in the vertical direction Z. As a result of this configuration, the medical applicator 10 is driven with, at least, 3 degrees of freedom.

The third driver which drives the medical applicator 10 with, at least, 1 degree of freedom is provided on the mount unit 40. The third driver moves the medical applicator 10 up and/or down to be, for example, closer to or farther away from the bed 21. The third driver moves the medical applicator 10 up and/or down in a radial direction of the arc shaped movement path of the mount unit 40. Additionally, the third driver rotates and/or rolls the medical applicator 10 about a vertical axis to drive the medical applicator 10 with an additional, at least, 2 degrees of freedom.

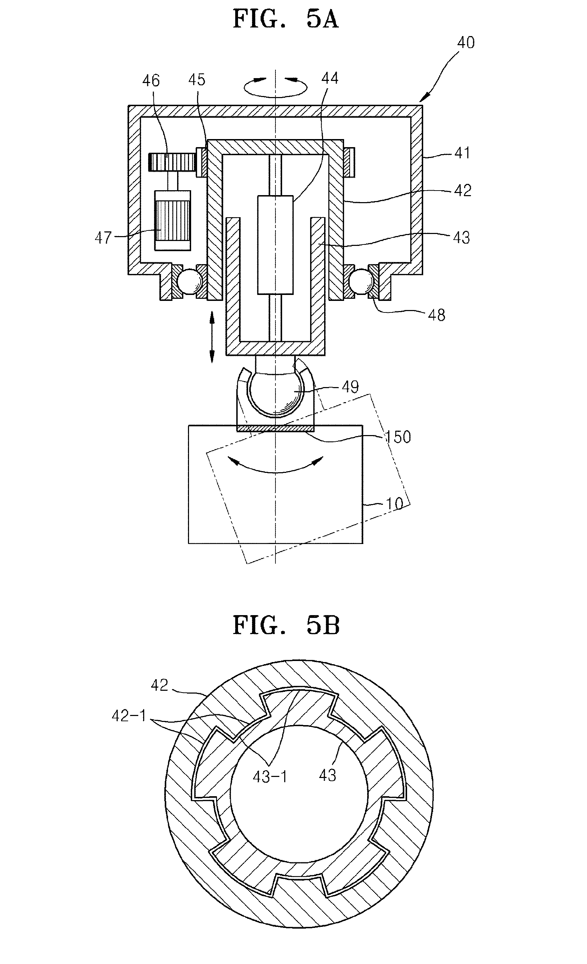

FIG. 5A is a cross-sectional view illustrating the third driver to raise and/or lower and rotate the medical applicator 10, according to an illustrative embodiment. Referring to FIG. 5A, a first member 42 and a second member 43 are provided in a housing 41. The first member 42 has, for example, a hollow cylindrical shape. In one configuration, the second member 43 is disposed, in part, in the first member 42 and includes, for example, a hollow cylindrical shape. The coupling unit 150 to which the medical applicator 10 is coupled to is provided at one end portion of the second member 43. The first member 42 and the second member 43 may be connected to each other through a linear actuator, such as a prismatic actuator 44, which provides a linear reciprocating force. The medical applicator 10 coupled to the coupling unit 150 may move up and/or down to be closer to or farther away from the bed 21 by driving the prismatic actuator 44 to raise and/or lower the second member 43 relative to the first member 42.

In this structure, the medical applicator 10 is positioned above the organ to be examined and/or treated by moving the moving unit 30 in the longitudinal direction X of the bed 21 and moving the mount unit 40 in the width direction Y. The medical applicator 10 is then moved down to an appropriate height for examination/treatment.

Referring back to FIG. 5A, in order to rotate the medical applicator 10 about the vertical axis, the third driver may further include a first gear 45 provided on the first member 42, and a rotary motor 47 including a second gear 46 engaged with the first gear 45. The rotary motor 47 is fixed to the housing 41 of the mount unit 40. The first member 42 is rotatably supported on the housing 41 via a bearing 48. When the rotary motor 47 rotates, the first member 42 also rotates about the vertical axis. A rotational force of the first member 42 may be transmitted through the prismatic actuator 44 to the second member 43 and, thus, the second member 43 also rotates. Alternatively, referring to FIG. 5B, a first uneven portion 42-1, which extends in an axial direction and includes protrusions and recesses may be formed on an inner circumferential surface of the first member 42. The recesses are alternately formed in a circumferential direction. A second uneven portion 43-1 has a shape conforming to that of the first uneven portion 42-1 and is formed on an outer circumferential surface of the second member 43. Accordingly, the first member 42 and the second member 43 rotate together, and the second member 43 rises and falls relative to the first member 42.

A configuration to raise the second member 43 relative to the first member 42 is not limited thereto. For example, although not shown in FIGS. 5A and 5B, a motor which rotates a lead screw may be provided on the first member 42. The lead screw may extend in the up-and-down direction, coupling projections meshing with helical grooves of the lead screw may be provided on the second member 43, and the second member 43 may move up and/or down by rotating the lead screw forward or backward.

Referring back to FIG. 5A, the second member 43 and the coupling unit 150 are connected to each other through a ball joint unit 49. In this structure, the medical applicator 10 may have, at least, passive 2 degrees of freedom, that is, yaw and pitch about the vertical axis. Because the medical applicator 10 yaws and pitches along the patient's body line, the medical applicator 10 may be kept in contact with the patient's body. Also, the medical applicator 10 may be kept in contact with the patient's body in response to movements while breathing of a patient's torso or abdomen.

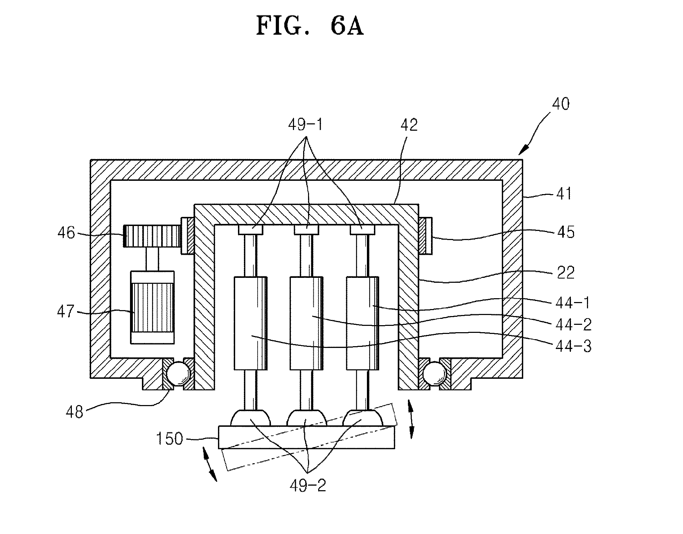

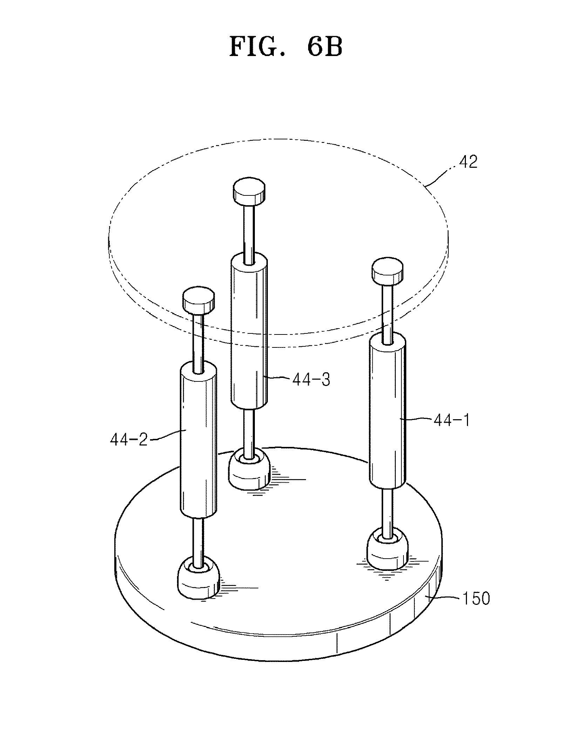

FIG. 6A is a cross-sectional view illustrating the third driver to actively roll, yaw, and pitch the medical applicator 10, according to another illustrative embodiment. FIG. 6B is a perspective view illustrating an arrangement of prismatic actuators 44-1, 44-2, and 44-3 of FIG. 6A.

Referring to FIG. 6A, the first member 42 is provided in the housing 41. The first member 42 includes, for example, a hollow cylindrical shape that extends in a radial direction of the arc shaped movement path of the moving unit 30. The first member 42 includes the first gear 45. The housing 41 includes the rotary motor 47 and the second gear 46, which is engaged with the first gear 45. The first member 42 is rotatably supported in the housing 41 via the bearing 48. When the rotary motor 47 rotates, the first member 42 rotates relative to the housing 41.

The coupling unit 150 is connected to the first member 42 through three linear actuators, that is, the prismatic actuators 44-1, 44-2, and 44-3. The first member 42 supports first ends of the prismatic actuators 44-1, 44-2, and 44-3. In one example, the first ends of the prismatic actuators 44-1, 44-2, and 44-3 are fixed to the first member 42. Second ends of the prismatic actuators 44-1, 44-2, and 44-3 are connected to the coupling unit 150 through three ball joint units 49-2. Referring to FIG. 6B, the prismatic actuators 44-1, 44-2, and 44-3 may be arranged at, for example, about 120° intervals.

In the above structure, when the rotary motor 47 rotates, the first member 42 rotates and the coupling unit 150 connected to the first member 42 also rotates due to the prismatic actuators 44-1, 44-2, and 44-3. Accordingly, rolling may be performed. The coupling unit 150 may move up and/or down relative to the first member 42 by equalizing operation distances between the prismatic actuators 44-1, 44-2, and 44-3. Also, the coupling unit 150 may yaw and pitch in an arbitrary direction by differentiating operation distances between the prismatic actuators 44-1, 44-2, and 44-3.

In one illustrative example, the medical applicator 10 may be positioned over the patient's organ or body part by moving the moving unit 30 in the longitudinal direction X of the bed 21, and moving the mount unit 40 in the width direction Y. Then, the medical applicator 10 may be lowered to an appropriate position for examination/treatment.

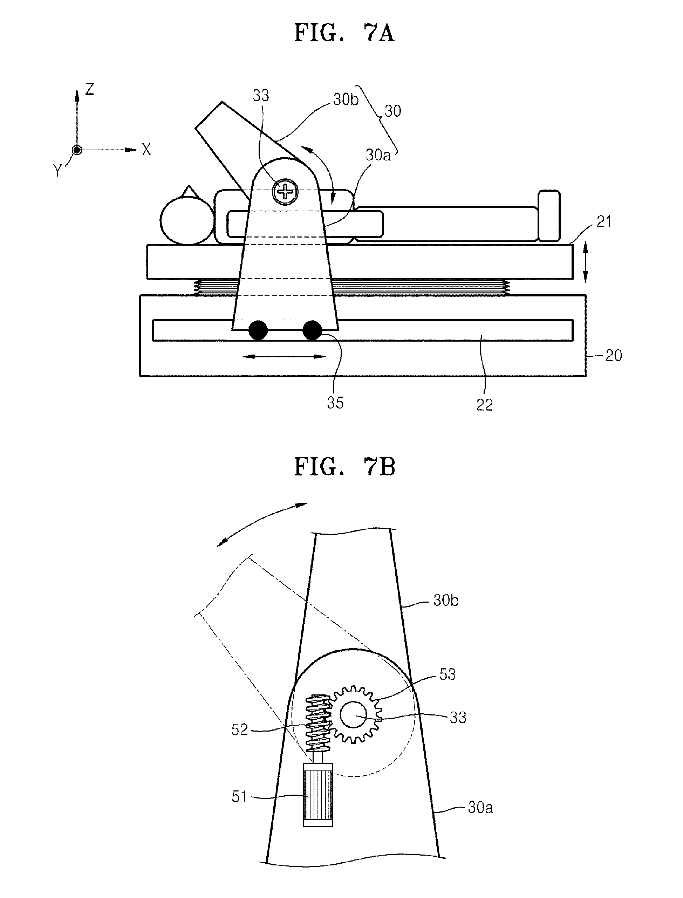

At least a portion of the moving unit 30 may pivot in the longitudinal direction X. A portion of the moving unit 30, including the movement path of the mount unit 40, may pivot in the longitudinal direction X. FIG. 7A is a side view illustrating the examination/treatment station 1, according to another illustrative embodiment. Referring to FIG. 7A, the moving unit 30 includes a support unit 30a and an extension unit 30b. The support unit 30a is supported on the base 20 and moves in the longitudinal direction X of the bed 21. The support unit 30a is connected to the first driver disposed on the base 20. For example, the support unit 30a is connected to the slider 120 of the linear motor 100 of FIG. 3. The extension unit 30b extends upward and crosses a space above the bed 21 in the width direction Y. The mount unit 40 of FIG. 3 may be provided on the extension unit 30b so that the mount unit 40 moves in the width direction Y.

The extension unit 30b may be substantially arc shaped in order to move the mount unit 40 in the width direction Y along the arc shaped movement path. For example, referring to FIG. 4, the second driver including the linear motor 200, which includes the linear guides 211 and 212 and the slider 220, may be disposed on the extension unit 30b.

The extension unit 30b is connected to the support unit 30a to pivot in the longitudinal direction X. Referring to FIG. 7B, the extension unit 30b may be connected to the support unit 30a to pivot about a pivotal axis 33. The pivotal axis 33 may be, for example, an axis in the width direction Y. As illustrated in FIG. 7B, a pivotal structure for pivoting the extension unit 30b about the pivotal axis 33 may include a combination of a worm 52 that is rotated by a rotary motor 51 provided on the support unit 30a and a worm gear 53 that is provided on the extension unit 30b. Alternatively, a combination of a crank arm, disposed on the extension unit 30b, and a linear actuator, connected to one end portion of the crank arm and supported on the support unit 30a, may be used as the pivotal structure.

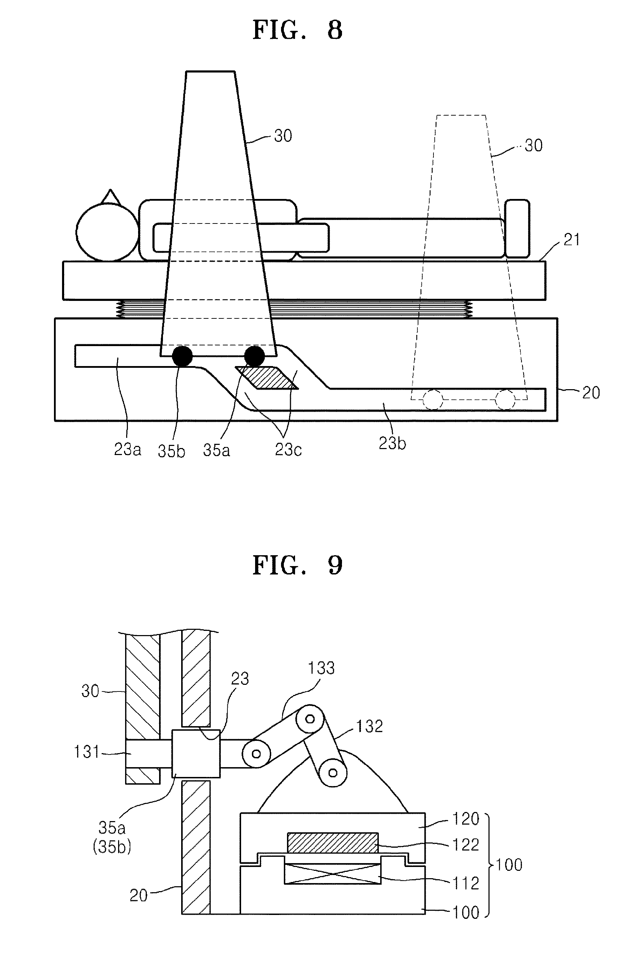

In one configuration, a portion of the movement path of the moving unit 30 is not parallel to the bed 21. That is, the portion of the movement path may be non-horizontal. For example, the patient's upper body is generally thicker than the patient's lower body. The movement path of the moving unit 30 may be configured such that the moving unit 30 moves down in the vertical direction Z when the moving unit 30 moves from an area corresponding to the patient's upper body to an area corresponding to the patient's lower body, and moves up in the vertical direction Z when the moving unit 30 moves from the area corresponding to the patient's lower body to the area corresponding to the patient's upper body. Due to the movement path, because a movement distance by which the medical applicator 10 moves to access the patient's body part to be examines and/or treated is reduced, the medical applicator 10 can easily access the body part to be examined and/or treated. FIG. 8 is a side view illustrating the examination/treatment station 1 in which the moving unit 30 moves up and/or down in the vertical direction Z while moving in the longitudinal direction X, according to another illustrative embodiment. FIG. 9 is a cross-sectional view illustrating the first driver to move the moving unit 30 in the longitudinal direction X and raising and/or lowering the moving unit 30 in the vertical direction Z of the examination/treatment station 1 of FIG. 8, according to an illustrative embodiment.

Referring to FIG. 8, a guide path 23 that defines a first path 23a corresponding to the patient's upper body and a second path 23b corresponding to the patient's lower body are formed inside the base 20. The first path 23a and the second path 23b may be connected to each other by third paths 23c that are inclined. The moving unit 30 may include guide rollers 35a and 35b that are inserted into the guide path 23. To raise and/or lower the moving unit 30, while maintaining the moving unit 30 in a horizontal state, the third paths 23c may be one pair of parallel paths along which the guide rollers 35a and 35b are respectively guided.

Referring to FIG. 9, the guide rollers 35a and 35b are rotatably supported on a rotational shaft 131 disposed on the moving unit 30. A first pivotal arm 132 is pivotably connected to the slider 120 of the linear motor 100. One end portion and the other end portion of a second pivotal arm 133 are respectively pivotably connected to the first pivotal arm 132 and the rotational shaft 131. As the slider 120 moves in the longitudinal direction X as a result of an electromagnetic force generated between the magnet 121 and the coil 112, the moving unit 30 is guided along the guide path 23 to move in the longitudinal direction X. For example, when the moving unit 30 transitions from the first path 23a to the third paths 23c, the guide rollers 35a and 35b are guided along the third paths 23c, which are inclined downward, and the moving unit 30 falls toward the second path 23b. In this process, because the second pivotal arm 133 and the first pivotal arm 132 pivot relative to the rotational shaft 131 and the slider 120, the rotational shaft 131 and the slider 120 may remain in continuous connection with each other. Also, because the rotational shaft 131 is kept in a horizontal state even when the guide rollers 35a and 35b pass through the third paths 23c, the moving unit 30 may maintain a horizontal state while moving downward from the first path 23a to the second path 23b via the third paths 23c. The moving unit 30 may also maintain a horizontal state while moving upward from the second path 23b to the first path 23a through the third paths 23c.

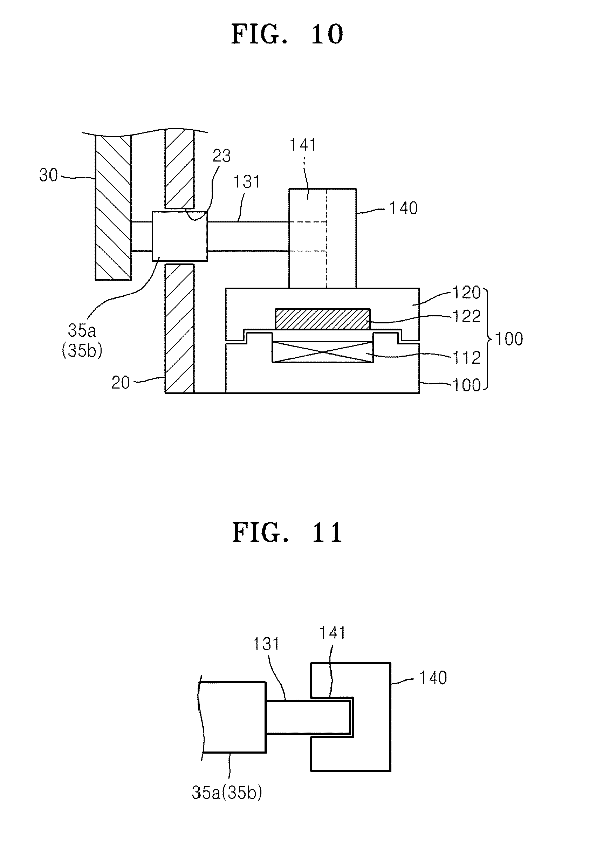

The first driver to raise and/or lower the moving unit 30 in the vertical direction Z is not limited to that shown in FIG. 9. For example, FIGS. 10 and 11 are cross-sectional views illustrating the first driver to raise and/or lower the moving unit 30 of the examination/treatment station 1, according to another illustrative embodiment. Referring to FIGS. 10 and 11, a connection member 140 having a "U"-shape and including an opening 141, which is open in the width direction Y, is disposed on the slider 120. The connection member 140 extends upward. The rotational shaft 131 of the guide rollers 35a and 35b is inserted into the opening 141. In this configuration, because the rotational shaft 131 moves up and/or down in the opening 141 while the guide rollers 35a and 354b are guided along the third paths 23c, the moving unit 30 may move up and/or down.

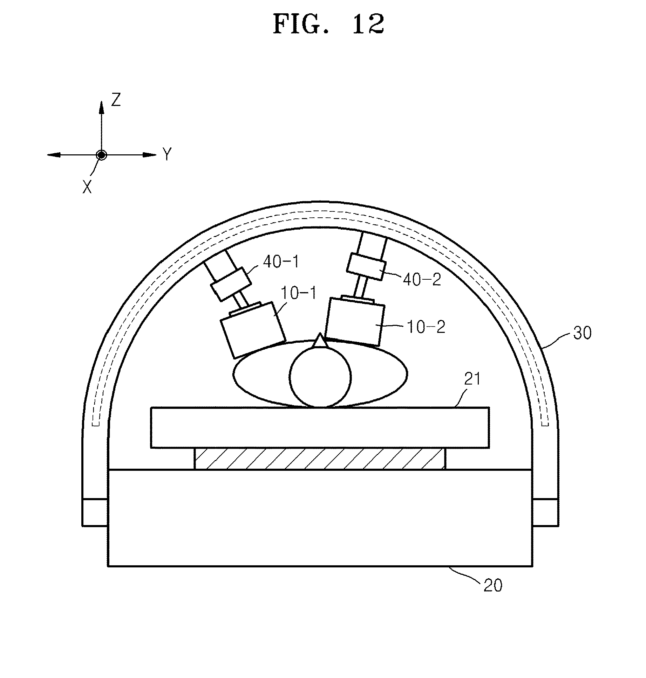

FIG. 12 is a front view illustrating the examination/treatment station 1, according to another illustrative embodiment. Referring to FIG. 12, two medical applicators 10-1 and 10-2 are mounted on the moving unit 30. For example, two mount units 40-1 and 40-2 are provided on the moving unit 30 to move in the width direction Y, and the medical applicators 10-1 and 10-2 are respectively connected to the mount units 40-1 and 40-2. In one configuration, the mount units 40-1 and 40-2 move together in the width direction Y. The mount units 40-1 and 40-2 are mounted on the slider 220 of the linear motor 200 of FIG. 4. Alternatively, in another configuration, the mount unit 40-1 moves independently from the movement of the mount unit 40-2 in the width direction Y. For this other configuration, two sliders 220 may be respectively provided on the linear guides 211 and 212 of the linear motor 200 of FIG. 4, and the mount units 40-1 and 40-2 may be respectively mounted on the two sliders 220. Alternatively, two linear motors 200 to respectively move the two mount units 40-1 and 40-2 in the width direction Y may be provided. The third driver would drive the medical applicators 10-1 and 10-2 with, at least, 1 degree of freedom. In this structure, two organs or two of the patient's body parts under testing and/or examination may be simultaneously examined/treated, or examination and treatment may be simultaneously performed on one organ or body part using the two medical applicators 10-1 and 10-2 having different purposes or the same purpose. Although not shown in FIG. 12, three or more medical applicators 10 may be mounted, if necessary.

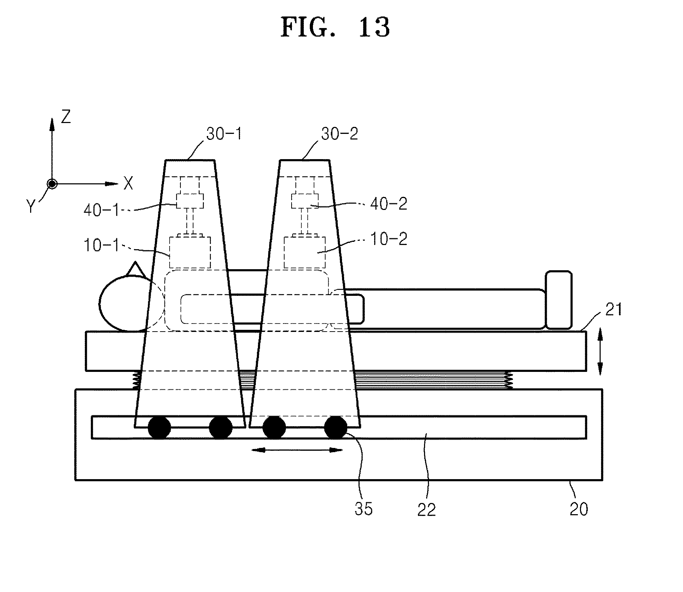

FIG. 13 is a side view illustrating the examination/treatment station 1, according to another illustrative embodiment. Referring to FIG. 13, two moving units 30-1 and 30-2 are provided on the base 20 to move in the longitudinal direction X. The two moving units 30-1 and 30-2 may independently move or may move together in the longitudinal direction X while maintaining an interval therebetween. The moving units 30-1 and 30-2 may be moved in the longitudinal direction X by, for example, the first driver of FIG. 3. For example, two sliders 120 may be disposed on the linear guide 110 that extends in the longitudinal direction X, and the moving units 30-1 and 30-2 may be respectively connected to the sliders 120. Alternatively, the moving units 30-1 and 30-2 may be respectively connected to two sliders 120, which are respectively disposed on two linear guides 110. The first driver to move the moving units 30-1 and 30-2 in the longitudinal direction X is not limited thereto. That is, the first driver may use a belt or a wire, a lead screw, or the like as desired. The medical applicators 10-1 and 10-2 are mounted on the moving units 30-1 and 30-2, respectively. For example, the mount units 40-1 and 40-2 may be provided on the moving units 30-1 and 30-2, respectively, to move in the width direction Y. The medical applicators 10-1 and 10-2 may be respectively coupled to the mount units 40-1 and 40-2, respectively. For example, the second driver of FIG. 4 may be used in order to move the mount units 40-1 and 40-2 in the width direction Y. The third driver would drive the medical applicators 10-1 and 10-2 with, at least, 1 degree of freedom. In this structure, two organs or body parts may be simultaneously examined/treated, or examination and treatment may be simultaneously performed on one organ or body part using the two medical applicators 10-1 and 10-2. The two medical applicators 10-1 and 10-2 may be configured to execute either same or different types of examination and/or treatments. Alternatively, two or more medical applicators may move on each of the moving units 30-1 and 30-2, and three or more moving units may be provided on the base 20 to move in the longitudinal direction X.

According to the medical robot system, the moving unit 30 on which the medical applicator 10 is mounted is integrally formed with the base 20 including the bed 21. That is, the first driver to move the moving unit 30 is integrally formed with the base 20. As such, the medical robot system is compact compared to a conventional medical system with a driving device to enable the medical applicator 10 to access an organ or a patient's body part, for example, a robot arm.

Because a conventional medical device is developed according to each operational purpose, a plurality of medical devices needs to be provided according to each operational purpose. In this case, a large space to install a plurality of medical devices is required. According to the medical robot system of the present configurations described above, however, the medical applicator 10 appropriate to each operational purpose may be mounted on the mount unit 40, and thus various medical operations may be performed through an integrated or a single robot system.

According to a conventional medical device in which the medical applicator 10 is mounted at a fixed position under the bed 21, the medical applicator 10 may access the organ or patient's body part only when the patient moves. By contrast, according to an illustrative example of the medical robot system of the present configurations, the first driver and the second driver move the medical applicator 10 to access the organ or body part of the patient who lies face up or down on the bed 21. Also, because the medical applicator 10 may use the third driver to access the patient's organ or body part adjacent to the patient's organ or body part in variety of angles and positions, examination/treatment may be effectively performed.

Various examination/treatment tools may be applied to the medical applicator 10. For example, the examination tool may be a needle or a surgical knife for a biopsy, a two-dimensional/three-dimensional (2D/3D) ultrasound probe, a laparoscopic camera, or an endoscopic camera. The treatment tool may be a non-invasive energy transmission device including an ultrasound treatment tool such as a high-intensity focused ultrasound (HIFU) transducer, a laser generator for treatment, or a radiofrequency ablation (RFA) probe. Alternatively, the treatment tool may be a minimally invasive surgical tool such as a multi-joint surgical tool for laparoscopy or a multi-joint surgical tool for single-port surgery.

The medical applicator 10 may be an ultrasound medical tool including a HIFU generator. HIFU treatment has advantages in that a type of non-invasive energy transmission, such as ultrasound, is used and lesions inside the body may be treated without using knifes or needles. HIFU treatment applies ultrasound to a specific portion inside the body where the lesion exists at an intensity, of about, a hundred thousand times greater than that of ultrasound intensity used for examination. The ultrasound applied to the lesion in the body is transformed into thermal energy and instantly increases a temperature of the specific portion inside the body where the lesion exists, thereby destroying the lesion through coagulation necrosis. Because the temperature of the specific portion inside the body where the lesion exists is instantly increased, heat may be prevented from spreading beyond the specific portion to which the ultrasound is applied and only the specific portion to which the ultrasound is applied may be effectively removed.

The HIFU treatment may use a transducer that receives an electrical signal and generates and applies ultrasound for treatment. The HIFU treatment may use an ultra-multi-element transducer to obtain a HIFU treatment effect. The ultrasound medical tool may include a transducer for treatment and an ultrasound probe to detect a position of a diseased organ or body part. For example, an image of a diseased organ or body part in an abdominal cavity may be displayed on the display 2-2 of the control station 2 after being obtained through the ultrasound probe. Consequently, the operator may apply high-intensity ultrasound output from the transducer to the diseased part while simultaneously viewing the image.

A position of the organ or body part may change during treatment due to the patient's respiration, heartbeat, or movement. When the medical applicator 10 is mounted at a fixed position, efficiency of the treatment may be reduced because of a failure to adjust to the change in the position of the organ or body part during treatment, resulting in unintentional damage to tissue other than that of the organ or body part. An ultrasound probe detects position information of the organ or body part detected and transmits the detected position, in real time, to the controller 2-1 of the control station 2. The controller 2-1 may effectively treat only the organ or body part by moving the medical applicator 10 in response to the position information of the organ or body part. According to the medical robot system, the medical applicator 10 is coupled to the mount unit 40, and the second driver to drive the medical applicator 10, with at least, 1 degree of freedom is disposed on the mount unit 40 to which the medical applicator 10 is coupled. Hence, the controller 2-1 may actively control the second driver in response to a change in the position of the organ or body part by generating a control signal to control the second driver based on changes in the position of the organ or body part. For example, while breathing, when the patient's chest or the abdomen moves up or down, the controller 2-1 drives the first driver to move the medical applicator 10 up or down, such that the transducer tracks and applies HIFU to the diseased part.

According to the mount unit 40 including the third driver of FIG. 6A, because the medical applicator 10 may, for example, roll, and yaw and pitch in an arbitrary direction, the medical applicator 10 is precisely driven in response to a change in the position of the diseased part.

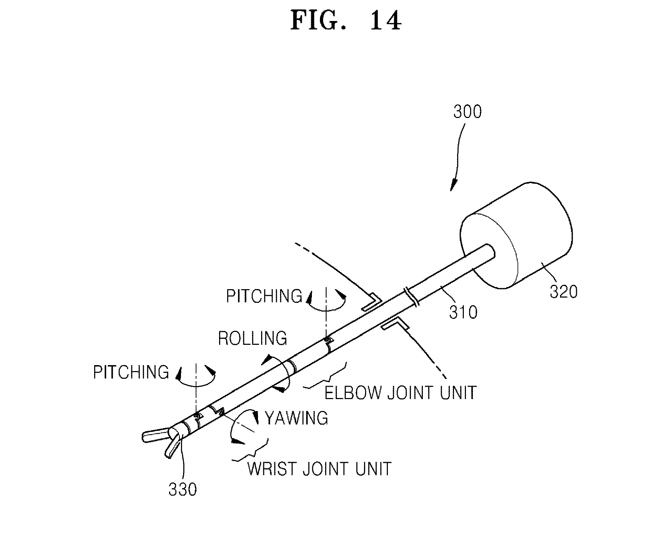

FIG. 14 is a perspective view illustrating a surgical applicator 300 to perform minimally invasive surgery as an example of the medical applicator 10, according to an illustrative embodiment. Referring to FIG. 14, the surgical applicator 300 includes a surgical arm 310 and a head unit 320. In order to access the diseased part, the surgical arm 310 may have, for example, a long rod shape inserted into the patient's abdominal cavity or joint. A surgical tool 330 to perform a surgical operation, such as incision or suture, according to the operator's manipulation is mounted on an end portion of the surgical arm 310. Examples of the surgical tool 330 include, but are not limited to, a surgical knife, surgical forceps, surgical scissors, and a cautery, which burns or cuts the diseased part using electrical energy or thermal energy. In order to perform various surgical operations, at least one joint unit with, at least, 1 degree of freedom sufficient may be disposed on the surgical arm 310. Examples of the joint unit include, but are not limited to, a wrist joint unit adjacent to the surgical tool 330 and an elbow joint unit spaced apart from the wrist joint unit. The wrist joint unit may be, for example, a joint unit, which may pitch and/or yaw. The elbow joint unit may be, for example, a joint unit that may pitch and/or roll. The head unit 320 includes a driving device to drive the joint unit and the surgical tool 330. The head unit 320 is connected to the controller 2-1 by being coupled to the coupling unit 150 disposed on the mount unit 40.

According to the examination/treatment station 1 of FIG. 12 or 13, a laparoscopic camera and the surgical applicator 300 of FIG. 14 may be mounted on the mount units 40-1 and 40-2, respectively. An operator may perform a surgical operation by operating the surgical applicator 300 using the manipulation unit 2-3 while viewing the display 2-2 to watch the organ or patient's body part through the laparoscopic camera and an image of a treatment procedure using the surgical applicator 300.

As described above, the first driver, the second driver, and/or the third driver may include a computing system or computer such as a microprocessor that is electrically connected with a bus, a user interface, and a memory controller. It may further include a flash memory device. The flash memory device may store N-bit data via the memory controller. The N-bit data is processed or will be processed by the microprocessor and N may be 1 or an integer greater than 1. Where the computing system or computer is a mobile apparatus, a battery may be additionally provided to supply operation voltage of the computing system or computer. It will be apparent to those of ordinary skill in the art that the computing system or computer may further include an application chipset, a camera image processor (CIS), a mobile Dynamic Random Access Memory (DRAM), and the like. The memory controller and the flash memory device may constitute a solid state drive/disk (SSD) that uses a non-volatile memory to store data.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A medical apparatus, comprising:
a moving unit supported on a base and configured to move in a longitudinal direction of a bed on the base and extend in a width direction of the bed; and
a mount unit on the moving unit and configured to move in the width direction and comprising a medical applicator mounted thereon.

2. The medical apparatus of claim 1, wherein the mount unit is configured to move through an arc shaped path of the moving unit in the width direction of the bed.

3. The medical apparatus of claim 1 or 2, wherein the moving unit comprises:
a support unit supported on the base and configured to move in the longitudinal direction of the bed, and
an extension unit extending in the width direction of the bed and configured to support the mount unit to move the mount unit in the width direction of the bed,
wherein the extension unit is connected to the support unit and pivots in the longitudinal direction of the bed.

4. The medical apparatus of any of the preceding claims, wherein the mount unit comprises
a coupling unit coupling to the medical applicator, and
a driver configured to drive the coupling unit with at least one degree of freedom.

5. The medical apparatus of claim 4, wherein the driver is configured to move the coupling unit up and/or down relative to the moving unit, in particular in a radial direction of the arc shaped movement path.

6. The medical apparatus of claim 4 or 5, wherein the driver is configured to rotate the coupling unit about a vertical axis.

7. The medical apparatus of any of claims 4-6, wherein the coupling unit is connected to the driver via a ball joint unit.

8. The medical apparatus of any of claims 4-7, wherein the driver comprises:
a first member rotatably coupled to a housing of the mount unit, and
three linear actuators which are configured to couple the first member and the coupling unit and which are radially arranged,
wherein the three linear actuators are each connected to the coupling unit via a ball joint unit.

9. The medical apparatus of any of claims 4-7, wherein the driver comprises
a first gear configured on the first member,
a rotary motor comprising a second gear engaged with the first gear, and
a second member partly disposed in the first member,
wherein the first member is rotatably supported on a housing of the mount unit via a bearing.

10. The medical apparatus of claim 9, wherein when the rotary motor rotates, the first member rotates about a vertical axis and a rotational force of the first member is transmitted through a prismatic actuator to the second member to enable the second member to rotate.

11. The medical apparatus of claim 9 or 10, wherein the first member and the second member rotate together, and the second member rises and falls relative to the first member.

12. The medical apparatus of any one of the preceding claims, wherein the medical applicator is releasably mounted on the mount unit.

13. The medical apparatus of any one of the preceding claims, wherein the medical applicator is configured for performing a HIFU treatment.

14. The medical apparatus of any of the preceding claims , wherein the moving unit is configured to move in the longitudinal direction along a movement path of which a portion is non-horizontal.

15. The medical apparatus of claim 14, wherein the movement path of the moving unit comprises first, second and third paths,
wherein the first and second paths correspond to an upper body and a lower body of a patient, respectively, and are of different heights with respect to the bed, and
wherein the third path is inclined and connects the first and second paths.
